Europäisches Patentamt

European Patent Office

Office européen des brevets

(19)

(11) Veröffentlichungsnummer : **0 073 052**
**B1**

(12) **EUROPÄISCHE PATENTSCHRIFT**

(45) Veröffentlichungstag der Patentschrift :
28.11.84

(21) Anmeldenummer : **82107697.3**

(22) Anmeldetag : **23.08.82**

(51) Int. Cl.$^3$ : **C 07 C149/243, C 07 B 19/00**

(54) Verfahren zur Trennung des Racemats S-(Carboxymethyl)-(RS)-cystein (A).

(30) Priorität : 28.08.81 DE 3134042

(43) Veröffentlichungstag der Anmeldung :
02.03.83 Patentblatt 83/09

(45) Bekanntmachung des Hinweises auf die Patenterteilung : 28.11.84 Patentblatt 84/48

(84) Benannte Vertragsstaaten :
AT BE CH DE FR GB IT LI NL SE

(56) Entgegenhaltungen :
AT-B- 320 607
DE-A- 2 653 332
DE-B- 2 045 998
DE-B- 2 362 687
THE JOURNAL OF ORGANIC CHEMISTRY, Vol. 16,
No. 5, 1951, MARVIN D. ARMSTRONG et al.,
"Thioether Derivatives of Cysteine and Homocysteine", Seiten 749-753

(73) Patentinhaber : Degussa Aktiengesellschaft
Weissfrauenstrasse 9
D-6000 Frankfurt am Main 1 (DE)

(72) Erfinder : Bethge, Horst
Schwalbenstrasse 2a
D-6450 Hanau 1 (DE)
Erfinder : Kleemann, Axel, Dr.
Grelfenhagenstrasse 9
D-6450 Hanau 9 (DE)
Erfinder : Martens, Jürgen, Dr.
Hochstrasse 5
D-8755 Alzenau (DE)

EP 0 073 052 B1

**Beschreibung**

Die Erfindung betrifft ein Verfahren zur Trennung des Racemats S-(Carboxymethyl)-(R,S)-cystein, insbesondere zwecks Gewinnung von S-(Carboxymethyl)-(R)-cystein. Diese Substanz wird für pharmazeutische Zwecke benötigt und dient beispielsweise als Mucolyticum.

Es ist bekannt, S-(Carboxymethyl)-(R)-cystein herzustellen, indem (R)-Cystein — auch als L-Cystein bezeichnet — mit Chloressigsäure in alkalischem Medium umgesetzt wird (J. Org. Chem. 16 (1951), 749 bis 753).

Das hierfür als Ausgangssubstanz benötigte (R)-Cystein wird im allgemeinen aus keratinhaltigen Naturstoffen gewonnen. Hierzu werden diese hydrolysiert ; das freigesetzte (R,R)-Cystin wird abgetrennt und zum (R)-Cystein reduziert (Org. Synth. Vol. 5 (1925), 39 bis 41 ; DE-OS 26 53 332 ; J. Am. Chem. Soc. 52 (1930), 4500). Geeignete Naturstoffe stehen jedoch nur in begrenztem Umfang zur Verfügung.

Bei der synthetischen Herstellung des Cysteins, beispielsweise aus in 2-Stellung substituierten Thiazolinen-(3) über die entsprechenden Thiazolidin-4-carbonitrile, entsteht das Racemat (R,S)-Cystein (DE-OS 26 45 748). Es ist bekannt, aus diesem das (R)-Cystein zu gewinnen, indem das (R,S)-Cystein mit Dicyandiamid zur (R,S)-2-Guanidin-1,3-thiazolidin-4-carbonsäure umgesetzt wird, aus dieser mit Hilfe des Kupferkomplexsalzes der (R)-Asparaginsäure die (R)-2-Guanidin-1,3-thiazolidin-4-carbonsäure abgetrennt wird und schließlich aus dieser das (R)-Cystein abgespalten wird (DE-AS 17 95 021). Dieses Verfahren zur Gewinnung des (R)-Cysteins ist so umständlich und aufwendig, daß es für eine Anwendung im technischen Maßstab ungeeignet ist.

Es ist nun gefunden worden, das Racemat S-(Carboxymethyl)-(R,S)-cystein mittels der optischen Isomeren des 2-Amino-1-phenyl-propan-1-ols zu trennen. Während man bei den bisherigen Verfahren zunächst (R)-Cystein, gegebenenfalls durch die umständliche Trennung des Racemats (R,S)-Cystein, gewinnt und dann das (R)-Cystein zum S-(Carboxymethyl)-(R)-cystein umsetzt, wird nunmehr zunächst das (R,S)-Cystein zum S-(Carboxymethyl)-(R,S)-cystein umgesetzt und dann dieses Racemat getrennt. Diese Trennung ist auf einfache Weise durchführbar und ergibt die optischen Isomeren des S-(Carboxymethyl)-cysteins bei hohen Ausbeuten in ausgezeichneter optischer und chemischer Reinheit.

Das S-(Carboxymethyl)-(R,S)-cystein wird aus dem (R,S)-Cystein in gleicher und bekannter Weise erzeugt wie das S-(Carboxymethyl)-(R)-cystein aus dem (R)-Cystein, nämlich beispielsweise durch Umsetzung mittels Chloressigsäure in alkalischem Medium nach dem in J. Org. Chem. 16 (1951), 749 bis 753, angegebenen Verfahren.

Erfindungsgemäß wird das S-(Carboxymethyl)-(R)-cystein mittels (1R,2S)-2-Amino-1-phenyl-propan-1-ol und das S-(Carboxymethyl)-(S)-cystein mittels (1S,2R)-2-Amino-1-phenyl-propan-1-ol aus dem Racemat abgetrennt. Die sich bildenden Salze aus (1R,2S)-2-Amino-1-phenyl-propan-1-ol und S-(Carboxymethyl)-(R)-cystein sowie aus (1S,2R)-2-Amino-1-phenyl-propan-1-ol und S-(Carboxymethyl)-(S)-cystein sind bisher nicht beschrieben. Das Salz aus (1R,2S)-2-Amino-1-phenyl-propan-1-ol und S-(Carboxymethyl)-(R)-cystein ist erheblich weniger löslich als das dazu diastereomere Salz aus (1R,2S)-2-Amino-1-phenyl-propan-1-ol und S-(Carboxymethyl)-(S)-cystein ; das Salz aus (1S,2R)-2-Amino-1-phenyl-propan-1-ol und S-(Carboxymethyl)-(S)-cystein ist erheblich weniger löslich als das dazu diastereomere Salz aus (1S,2R)-2-Amino-1-phenyl-propan-1-ol und S-(Carboxymethyl)-(R)-cystein.

Zur Durchführung des erfindungsgemäßen Verfahrens wird in der bei Racemattrennungen üblichen Weise vorgegangen. Das Racemat S-(Carboxymethyl)-(R,S)-cystein wird in Gegenwart von Lösungsmitteln mit dem gewünschten optischen Isomeren des 2-Amino-1-phenyl-propan-1-ols zusammengebracht, und es werden dann die gebildeten diastereomeren Salze getrennt.

Die zueinander diastereomeren Salze zeigen in zahlreichen Lösungsmitteln ausreichend große Löslichkeitsunterschiede. Zu diesen Lösungsmitteln gehört beispielsweise Wasser. Vorzugsweise werden als Lösungsmittel primäre oder sekundäre Alkanole mit bis zu 6 Kohlenstoffatomen oder Äther verwendet und unter diesen Lösungsmittel insbesondere solche, die mit Wasser unbegrenzt mischbar sind. In Frage kommen beispielsweise Hexan-1-ol, Butan-1-ol, Methyl-tert.-butyläther, insbesondere Methanol, Äthanol, Propan-2-ol, Dioxan und Tetrahydrofuran. Die Lösungsmittel können auch in Gemischen untereinander oder in Gemischen mit Wasser angewendet werden, jedoch werden die Mischungen zweckmäßigerweise so gewählt, daß die Lösungsmittel nicht mehr als eine Phase bilden.

Das Racemat S-(Carboxymethyl)-(R,S)-cystein wie auch das betreffende optische Isomere des 2-Amino-1-phenyl-propan-1-ols können in fester Form oder als Aufschlämmung oder Lösung in dem Lösungsmittel eingesetzt werden. Das optische Isomere des 2-Amino-1-phenyl-propan-1-ols und das Racemat S-(Carboxymethyl)-(R,S)-cystein können zwar in beliebigen Mengenverhältnissen zueinander eingesetzt werden, es ist im allgemeinen jedoch zweckmäßig, je mol des Racemats nicht weniger als etwa 0,5 und nicht mehr als etwa 5,0 mol des optischen Isomeren zu nehmen. Vorzugsweise werden je mol des Racemats 0,9 bis 1,1, insbesondere 1,0 mol des optischen Isomeren angewendet. Es kann bei allen Temperaturen gearbeitet werden, bei denen die Lösungsmittel in flüssiger Form vorliegen.

Zur Trennung der diastereomeren Salze wird vorzugsweise in der bei einer fraktionierten Kristallisation üblichen Weise vorgegangen. Es wird die Mischung auf erhöhte Temperaturen, vorzugsweise auf Temperaturen nahe dem Siedepunkt, gebracht, so viel Lösungsmittel angewendet, daß alle Substanzen gelöst sind, und schließlich die Lösung zur Kristallisation abgekühlt.

Aus den anfallenden Salzen aus S-(Carboxymethyl)-(R)-cystein und (1R,2S)-2-Amino-1-phenyl-propan-1-ol oder S-(Carboxymethyl)-(S)-cystein und (1S,2R)-2-Amino-1-phenyl-propan-1-ol wird das betreffende S-(Carboxymethyl)-cystein freigesetzt, indem die Salze mit starker Säure, vorzugsweise starker Mineralsäure, wie Chlorwasserstoffsäure, behandelt werden.

Beispiele

Die gewonnenen optisch aktiven Substanzen wurden jeweils auf Ihren spezifischen Drehwert $[\alpha]_D^{20}$ untersucht. Dieser wird in Grad · cm³/dm · g angegeben. Prozentangaben bedeuten Gewichtsprozente.

A. Herstellung des S-(Carboxymethyl)-(R,S)-cysteins

Als Ausgangssubstanz diente (R,S)-Cystein-hydrochlorid, das nach dem Verfahren gemäß der DE-OS 26 45 748 hergestellt war. Es wurden 140 g (1 mol) dieser Substanz mit 160 g (4 mol) Natriumhydroxid in 1 000 ml Wasser gelöst. Dieser Lösung wurden zunächst 3 g Natrium-hydrogensulfid und dann im Verlauf von 45 Minuten 95 g (1 mol) Monochloressigsäure zugesetzt. Die Temperatur der Mischung wurde währenddessen auf 20 °C und danach 3 Stunden lang auf 20 bis 30 °C gehalten. Das Umsetzungsgemisch wurde schließlich durch Zugabe konzentrierter wäßriger Chlorwasserstoffsäure auf den pH-Wert 3,0 eingestellt. Hierbei schied sich das S-(Carboxymethyl)-(R,S)-cystein ab. Es wurde bei 10 °C abfiltriert und so lange mit Wasser gewaschen, bis es frei von Chlorionen war. Dann wurde es unter Unterdruck bei 150 °C getrocknet. Die Ausbeute betrug 173 g, entsprechend 97 %, bezogen auf das eingesetzte Cystein-hydrochlorid. Der Schmelzpunkt (Zersetzungspunkt) des S-(Carboxymethyl)-(R,S)-cysteins war 188 bis 192 °C

B. Trennung des Racemats S-(Carboxymethyl)-(R,S)-cystein

1. 50,0 g (0,28 mol) des nach A, gewonnenen Racemats S-(Carboxymethyl)-(R,S)-cystein wurden in 400 ml Methanol suspendiert, das 10 % Wasser enthielt. Dieser Suspension wurden im Verlauf einer Stunde 42,5 g (0,28 mol) (1R,2S)-2-Amino-1-phenyl-propan-1-ol zugesetzt. Die Mischung wurde währenddessen auf 40 bis 50 °C und weiter 30 Minuten lang unter Rückfluß auf Siedetemperatur gehalten, dann langsam auf 30 °C abgekühlt und filtriert. Der Rückstand wurde mit 200 ml wasserfreiem Methanol gewaschen und bei 50 °C und 25 mbar getrocknet. Die gewonnene Substanz war das Salz aus S-(Carboxymethyl)-(R)-cystein und (1R,2S)-2-Amino-1-phenyl-propan-1-ol. Die Ausbeute betrug 41,6 g, entsprechend 90 %, bezogen auf das in dem eingesetzten Racemat enthaltene S-(Carboxymethyl)-(R)-cystein. Der Schmelzpunkt des gewonnenen Salzes war 68 °C und der spezifische Drehwert − 38,3° (c = 1 in Wasser).

Das Salz wurde in 150 ml Wasser gelöst, und die Lösung wurde bei 25 °C mittels 2 n wäßriger Chlorwasserstoffsäure auf den pH-Wert 3,0 eingestellt. Hierbei wurde das S-(Carboxymethyl)-(R)-cystein ausgefällt. Es wurde abfiltriert, mit 100 ml kaltem Wasser gewaschen und dann bei 105 °C und 25 mbar getrocknet. Die Ausbeute betrug 21,7 g, entsprechend 96 %, bezogen auf das eingesetzte Salz. Der Schmelzpunkt (Zersetzungspunkt) des S-(Carboxymethyl)-(R)-cysteins war 190 bis 192 °C und der spezifische Drehwert − 34,5° (c = 10 in wäßriger Natriumhydroxidlösung, pH-Wert 6,0).

Das beim Abfiltrieren des S-(Carboxymethyl)-(R)-cysteins verbliebene Filtrat wurde mit 200 ml einer 50 %igen wäßrigen Natriumhydroxidlösung versetzt. Die Mischung wurde dreimal mit je 100 ml Methylenchlorid extrahiert. Die vereinigten Extrakte wurden mit Natriumsulfat getrocknet. Dann wurde das Methylenchlorid abgetrieben und der Rückstand in Methyl-tert.-butyl-äther umkristallisiert. Gewonnen wurden 18,6 g (1R,2S)-2-Amino-1-phenyl-propan-1-ol.

Das beim Abfiltrieren des Salzes aus S-(Carboxymethyl)-(R)-cystein und (1R,2S)-2-Amino-1-phenyl-propan-1-ol verbliebene Filtrat wurde zur Trockne eingedampft. Der Rückstand wurde in 200 ml Wasser aufgenommen, und die Mischung wurde mit 2 n wäßriger Chlorwasserstoffsäure auf den pH-Wert 3,0 eingestellt. Hierbei wurde S-(Carboxymethyl)-(S)-cystein mit geringen Anteilen S-(Carboxymethyl)-(R)-cystein abgeschieden. Die Substanz wurde abfiltriert, dann zunächst in einer Mischung aus Essigsäure und Essigsäureanhydrid erhitzt und nach Abtreiben der Essigsäure mit Chlorwasserstoffsäure behandelt. Hierbei wurde racemisches S-(Carboxymethyl)-cystein gewonnen.

Das beim Abfiltrieren des S-(Carboxymethyl)-(S)-cysteins verbliebene Filtrat wurde mit 200 ml 50 %iger wäßriger Natriumhydroxidlösung versetzt. Die Mischung wurde dreimal mit je 100 ml Methylenchlorid extrahiert. Die vereinigten Extrakte wurden mit Natriumsulfat getrocknet. Dann wurde das Methylenchlorid abgetrieben und der Rückstand in Methyl-tert.-butyläther umkristallisiert. Gewonnen wurden hierbei 23,1 g (1R,2S)-2-Amino-1-phenyl-propan-1-ol. Insgesamt wurden also 41,7 g, entsprechend 98 %, des eingesetzten (1R,2S)-2-Amino-1-phenyl-propan-1-ols zurückgewonnen.

2. Es wurde wie nach B.1. verfahren, jedoch wurden die Ausgangssubstanzen in 1 000 ml wasserfreiem Methanol suspendiert, und die Suspension wurde 2 Stunden lang unter Rühren auf 50 bis 60 °C gehalten. Gewonnen wurden 37,0 g, entsprechend 80 % Ausbeute, des Salzes aus S-(Carboxymethyl)-(R)-cystein und (1R,2S)-2-Amino-1-phenyl-propan-1-ol. Der Schmelzpunkt des Salzes war 68 °C und der Drehwert − 38,3° (c = 1 in Wasser). Aus diesem Salz wurde wie nach B.1. das S-(Carboxymethyl)-(R)-cystein gewonnen. Die Ausbeute betrug 19,3 g, entsprechend 96 %. Der Schmelzpunkt (Zersetzungspunkt) war 188 bis 191 °C und der Drehwert − 34,0° (c = 1 in wäßriger Natriumhydroxidlösung, pH-Wert = 6,0).

Das beim Abfiltrieren des Salzes aus S-(Carboxymethyl)-(R)-cystein und (1R,2S)-2-Amino-1-phenyl-propan-1-ol verbliebene Filtrat wurde mit 2 n wäßriger Chlorwasserstoffsäure auf den pH-Wert 3,0 eingestellt. Die hierbei abgeschiedene Substanz wurde abfiltriert, mit kaltem Wasser gewaschen und bei 105 °C und 25 mbar getrocknet. Sie bestand zu 79 % aus S-(Carboxymethyl)-(S)-cystein und zu 21 % aus S-(Carboxymethyl)-(R)-cystein. Gewonnen wurden 26,8 g, entsprechend 53,6 %, bezogen auf das eingesetzte S-(Carboxymethyl)-(R,S)-cystein. Der Drehwert der Substanz war + 20,0° (c = 10 in wäßriger Natriumhydroxidlösung, pH-Wert = 6,0).

3. Es wurde wie nach B.1. verfahren, jedoch wurde das S-(Carboxymethyl)-(R,S)-cystein in 1 000 ml wasserfreiem Methanol suspendiert. Gewonnen wurden 37,0 g, entsprechend 80 % Ausbeute, des Salzes aus S-(Carboxymethyl)-(R)-cystein und (1R,2S)-2-Amino-1-phenyl-propan-1-ol. Der Schmelzpunkt des Salzes war 69 °C und der Drehwert − 37,5° (c = 1 in Wasser). Die Elementaranalyse des Salzes ergab: C = 50,79 % (50,89 %); H = 6,80 % (6,71 %); N = 8,47 % (8,48 %); S = 9,78 % (9,71 %); (in Klammern die für $C_{14}H_{22}N_2O_5S$ berechneten Werte).

4. Es wurde wie nach B.1. verfahren, jedoch wurden die Ausgangssubstanzen in 800 ml Dioxan suspendiert, und dieser Suspension wurde bei Siedetemperatur so lange Wasser zugeführt, bis alle Substanzen gelöst waren. Die Lösung wurde im Verlauf von 2 Stunden auf 25 °C abgekühlt. Gewonnen wurden 36,0 g, entsprechend 78 % Ausbeute, des Salzes aus S-(Carboxymethyl)-(R)-cystein und (1R,1S)-2-Amino-1-phenyl-propan-1-ol. Der Schmelzpunkt war 68 °C und der Drehwert − 37,4° (c = 1 in Wasser).

5. Es wurde nach B.4. verfahren, jedoch wurden statt des Dioxans 800 ml Propan-2-ol eingesetzt. Die Ausbeute betrug 43,9 g, entsprechend 95 %. Der Schmelzpunkt war 67 °C und der Drehwert − 38,0° (c = 1 in Wasser).

6. Es wurde wie nach B.4. verfahren, jedoch wurden statt des Dioxans 200 ml Tetrahydrofuran eingesetzt. Die Ausbeute betrug 54,5 g, entsprechend 96 %. Der Schmelzpunkt war 67 °C und der Drehwert − 38,4° (c = 1 in Wasser).

7. Es wurde wie nach B.1. verfahren, jedoch wurde das S-(Carboxymethyl)-(R,S)-cystein in 100 ml Wasser suspendiert, dieser Suspension das (1R,2S)-2-Amino-1-phenyl-propan-1-ol zugesetzt, die Suspension auf 60 °C erwärmt und unter Unterdruck auf 105 g eingedampft und der Rückstand in 800 ml siedendem Methanol aufgenommen. Gewonnen wurden 37,4 g, entsprechend 81 % Ausbeute, des Salzes aus S-(Carboxymethyl)-(R)-cystein und (1R,2S)-2-Amino-1-phenyl-propan-1-ol. Der Schmelzpunkt war 68 °C und der Drehwert − 38,1° (c = 1 in Wasser).

8. Es wurde wie nach B.7. verfahren, jedoch wurden statt Methanol 800 ml Äthanol eingesetzt. Die Ausbeute betrug 37,0 g, entsprechend 80 %. Der Schmelzpunkt war 67 °C und der Drehwert − 37,8° (c = 1 in Wasser).

9. Es wurde wie nach B.2. verfahren, jedoch wurden 100 g (0,56 mol) S-(Carboxymethyl)-(R,S)-cystein und 124 g (0,82 mol) (1R,2S)-2-Amino-1-phenyl-propan-1-ol in 800 ml Methanol eingesetzt, das 2 % Wasser enthielt, die Mischung wurde eine Stunde lang unter Rückfluß auf Siedetemperatur gehalten und dann im Verlauf von 30 Minuten auf 30 °C abgekühlt. Gewonnen wurden 89,0 g, entsprechend 96 %, des Salzes aus S-(Carboxymethyl)-(R)-cystein und (1R,2S)-2-Amino-1-phenyl-propan-1-ol. Der Schmelzpunkt des Salzes war 67 °C und der Drehwert − 37,4° (c = 1 in Wasser).

10. Es wurde wie nach B.1. verfahren, jedoch wurden statt (1R,2S)-2-Amino-1-phenyl-propan-1-ol 42,5 g (0,28 mol) (1S,2R)-2-Amino-1-phenyl-propan-1-ol eingesetzt. Gewonnen wurde das Salz aus S-(Carboxymethyl)-(S)-cystein und (1S,2R)-2-Amino-1-phenyl-propan-1-ol. Die Ausbeute betrug 39,3 g, entsprechend 85 %, bezogen auf das in dem eingesetzten Racemat enthaltene S-(Carboxymethyl)-(S)-cystein. Die Elementaranalyse ergab: C = 50,97 % (50,89 %); H = 6,70 % (6,71 %); N = 8,25 % (8,48 %); S = 9,88 % (9,71 %) (in Klammern die für $C_{14}H_{22}N_2O_5S$ berechneten Werte). Aus dem gewonnenen Salz wurde wie nach B.1. das S-(Carboxymethyl)-(S)-cystein freigesetzt. Die Ausbeute betrug 20,4 g, entsprechend 96 %, bezogen auf das eingesetzte Salz. Der Schmelzpunkt (Zersetzungspunkt) des S-(Carboxymethyl)-(S)-cysteins war 189 bis 191 °C und der Drehwert + 34,2° (c = 10 in wäßriger Natriumhydroxidlösung, pH-Wert 6,0).

**Ansprüche**

1. Trennung des Racemats S-(Carboxymethyl)-(R,S)-cystein mittels der optischen Isomeren des 2-Amino-1-phenyl-propan-1-ols.

2. Salz aus S-(Carboxymethyl)-(R)-cystein und (1R,2S)-2-Amino-1-phenyl-propan-1-ol.

3. Salz aus S-(Carboxymethyl)-(S)-cystein und (1S,2R)-2-Amino-1-phenyl-propan-1-ol.

## Claims

1. Resolution of the racemate S-(carboxymethyl)-(R,S)-cystein using the optical isomers of 2-amino-1-phenyl-propan-1-ol.

2. Salt consisting of S-(carboxymethyl)-(R)-cystein and (1R,2S)-2-amino-1-phenyl-propan-1-ol.

3. Salt consisting of S-(carboxymethyl)-(S)-cystein and (1S,2R)-2-amino-1-phenyl-propan-1-ol.

## Revendications

1. Dédoublement de la S-(carboxyméthyl)-(R,S)-cystéine racémique, à l'aide des isomères optiques du 2-amino-1-phényl-propan-1-ol.

2. Sel formé de S-(carboxyméthyl)-(R)-cystéine et de (1R,2S)-2-amino-1-phényl-propan-1-ol.

3. Sel formé de S-(carboxyméthyl)-(S)-cystéine et de (1S,2R)-2-amino-1-phényl-propan-1-ol.